(19) **Europäisches Patentamt / European Patent Office / Office européen des brevets**

(11) **EP 3 369 418 B1**

(12) **EUROPEAN PATENT SPECIFICATION**

(45) Date of publication and mention of the grant of the patent:
**23.06.2021 Bulletin 2021/25**

(21) Application number: **16859986.8**

(22) Date of filing: **28.10.2016**

(51) Int Cl.:
*A61K 8/81* *(2006.01)*     *A61K 8/04* *(2006.01)*
*A61K 8/92* *(2006.01)*     *A61Q 19/00* *(2006.01)*
*A61K 8/31* *(2006.01)*     *A61K 8/37* *(2006.01)*
*A61K 8/73* *(2006.01)*     *A61K 8/88* *(2006.01)*
*A61K 8/891* *(2006.01)*

(86) International application number:
**PCT/JP2016/082139**

(87) International publication number:
**WO 2017/073758 (04.05.2017 Gazette 2017/18)**

(54) **COMPOSITION FOR CORRECTING PORES OF THE SKIN**

ZUSAMMENSETZUNG ZUR KORREKTUR DER POREN DER HAUT

COMPOSITION POUR CORRIGER LES PORES DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2015 JP 2015215124**

(43) Date of publication of application:
**05.09.2018 Bulletin 2018/36**

(73) Proprietor: **Shiseido Company, Ltd.**
**Chuo-ku,**
**Tokyo 104-0061 (JP)**

(72) Inventors:
• **HOSOKAWA, Kinya**
**Yokohama-shi**
**Kanagawa 224-8558 (JP)**

• **NISHIDA, Keita**
**Yokohama-shi**
**Kanagawa 224-8558 (JP)**

(74) Representative: **Ter Meer Steinmeister & Partner**
**Patentanwälte mbB**
**Nymphenburger Straße 4**
**80335 München (DE)**

(56) References cited:
EP-A1- 1 627 629        WO-A2-2011/068333
JP-A- H0 219 310        JP-A- H05 201 826
JP-A- 2004 210 655      JP-A- 2004 285 016
JP-A- 2007 197 446      JP-A- 2007 262 032
US-A- 5 223 559         US-A1- 2004 096 414
US-A1- 2015 110 841

## Description

[Technical Field]

The present invention relates to a composition, and more particularly, to a composition providing an impression during use similar to that of skin care cosmetics and a pore-correcting effect.

[Background Art]

Skin care and making-up are usually performed by conditioning the skin using skin care cosmetics such as a toning lotion, then correcting defects (color tones or irregularities) of the skin using a foundation, and as necessary further adding the intended color tones and seals to improve the appearance.

Therefore, a coloring agent or a powder having a high covering power is used less frequently for skin care cosmetics, whereas an inorganic powder having a high refractive index (covering power) such as titanium oxide and zinc oxide is commonly blended into makeup cosmetics such as a foundation in order to cover defects on the skin.

However, there has recently been a growing preference for bare skin mainly in young people, and these people also tend to refrain from using makeup cosmetics which even cover a unique translucent color of the bare skin.

[Prior Art Documents]

[Patent Literatures]

[Patent Literature 1] Japanese Unexamined Patent Publication No. H09-48723
[Patent Literature 2] Japanese Unexamined Patent Publication No. 2006-63032
[Patent Literature 3] Japanese Unexamined Patent Publication No. 3291195
[Patent Literature 4] Japanese Unexamined Patent Publication No. H10-203936
[Patent Literature 5] Japanese Unexamined Patent Publication No. H05-201826

[Summary of the Invention]

[Problem to be Solved by the Invention]

The present invention was made in view of the above-described problems of the conventional art, and an object is to provide a composition having like a skin care cosmetics appearance and an texture and correcting a skin irregularity such as pores without almost all using a powder having a high covering power

[Means to Solve the Problem]

To solve the above problem, the present inventors have conducted a study and consequently found out that dispersing a lipophilic porous powder and an oil in an aqueous medium can give like a skin care cosmetics appearance and an texture and provide a skin irregularity-correcting effect, leading to the completion of the present invention. That is, the composition of the present invention comprises:

an aqueous medium;
a lipophilic porous powder dispersed in the aqueous medium;
an oil having a viscosity of 100 mPa.s or less, the oil being kept by at least some of pores of the lipophilic porous powder; and
a dispersing agent for dispersing the lipophilic porous powder in the aqueous medium.

Furthermore, it is preferable that in the present invention the lipophilic porous powder comprises crosslinked polymethyl methacrylate as a main material.

In the present invention a maximum allowable oil absorption of the lipophilic porous powder is 100% by mass or more based on the powder. Furthermore, Oil absorption is measured by placing a sample onto a glass plate, dripping glyceryl tri-2-ethylhexanoate by a small amount and kneading by a palette knife until the hardness of the paste becomes smooth. Dripping weight/sample weight is set as oil absorption (%).

In the present invention the composition satisfies the following formula:

EP 3 369 418 B1

$$[(\text{Oil content (\% by mass) of composition})/(\text{Lipophilic porous powder content (\% by mass) of composition}) \square 100]/\text{Maximum oil absorption (\% by mass) of lipophilic porous powder} \square 1.$$

Furthermore, it is preferable in the present invention a particulate wax is blended into the composition. Furthermore, it is preferable in the present a viscosity of the composition is 100000 mPa.s or less at 25 °C. Furthermore, it is preferable the composition is for use in correcting pores.

Furthermore, correcting pores use of the composition is preferable.

The correcting pores method of the present invention is characterized that the composition is applied to skin.

Further, the composition according to the present invention can be obtained by adding a dispersing agent to an aqueous medium, dispersing a lipophilic porous powder to the aqueous medium, and then adding an oil component having viscosity of 100 mPa·s or less to the aqueous medium.

Further, a production method of the composition according to the present invention comprises: adding a dispersing agent to an aqueous medium; dispersing a lipophilic porous powder to the aqueous medium; and adding an oil component having viscosity of 100 mPa·s or less to the aqueous medium.

[Effect of the Invention]

According to the composition of the present invention, blending the lipophilic porous powder into the aqueous medium provides the following effect: when the composition is applied to the skin, the lipophilic porous powder falls into the depressed parts such as pores, and the light diffusion effect of powder make the depressed parts unnoticeable. Further, the lipophilic porous powder keeps the oil, whitening due to excessive light scattering is prevented, and moreover the oil seeping from the lipophilic porous powder gives a slippery effect when the composition is used, which can provide an excellent impression during use.

[Brief Description of the Drawings]

Hereinafter, embodiments of the present invention will be described in detail.

[Lipophilic porous powder]

As a lipophilic porous powder which is preferably used in the present invention, crosslinked polymethyl methacrylate, a nylon porous powder, or the like is preferably used. The material itself of the powder may be lipophilic or the powder may be made of a hydrophilic material but coated with a lipophilic substance, and in the case of coating, even the inside of pores of the material is preferably lipophilized.

The maximum allowable oil absorption of the lipophilic porous powder is 100% by mass or more based on the powder. The maximum allowable oil absorption is also closely related to the porous volume, and the porous form affects light diffusion properties. For these reasons, a maximum allowable oil absorption of less than 100% by mass provides an insufficient light diffusion effect and thus an insufficient pore-correcting effect.

[Oil]

As an oil which is preferably used in the present invention, a wide variety of oils such as a polar oil and a silicone oil can be used, but the viscosity thereof needs to be 100 mPa·s or less. A viscosity of more than 100 mPa·s makes it difficult for the lipophilic porous powder to keep the oil and it difficult to prevent whitening caused by the lipophilic porous powder.

Examples of the polar oil include ester oils such as glyceryl tri-2-ethylhexanoate, and particularly when crosslinked polymethyl methacrylate is used as the lipophilic porous powder, glyceryl tri-2-ethylhexanoate is preferable.

When the polar oil is used, the viscosity of the composition is kept relatively low, and impressions during use (wateriness, non-stickiness, and the like) similar to those of skin care compositions such as a essence serum and a milky lotion in particular can be obtained.

Examples of the non-polar oil include methyl polysiloxane, methylphenyl polysiloxane, and liquid paraffin, and when the non-polar oil is used, a relatively high viscosity can be obtained and thus the impressions during use similar to those of gel-form skin care compositions can be obtained.

The oil content satisfies the following formula:

[(Oil content (% by mass) of composition)/(Lipophilic porous powder content (% by mass) of composition) × 100]/Maximum oil absorption (% by mass) of lipophilic porous powder ≤ 1

When the oil content does not satisfy the above formula, no pore-correcting effect can be obtained. When the oil content is less than 0.1 times, a poor pore-correcting effect may be obtained.

Examples of oils include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glycerol di-2-heptyl undecanoate, trimethyrol propane tri-2-ethyl hexanoate, trimethyrol propane triisostearate, tetra-2-ethyl hexanoate pentaerythritol, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of silicone oils include chain polysiloxane (for example, dimethylpolysiloxane, methylphenylpolysiloxane, and diphenylpolysiloxane); cyclic polysiloxane (for example, octamethylcyclotetrasiloxane, decamethylcyclopentasiloxane, and dodecamethylcyclohexasiloxane), silicone resins having a three dimensional network structure, silicone rubber, various modified polysiloxanes (for example, amino-modified polysiloxane, polyether-modified polysiloxane, alkyl-modified polysiloxane, and fluorine-modified polysiloxane), and acrylic silicone.

The oil which is preferably used in the present invention preferably has a viscosity less than 100mPa·s as a whole of the oil..

[Dispersing agent]

In the present invention, carboxylic acid polymers such as a carboxy vinyl polymer, sodium polyacrylate, and an acrylic acid/alkyl methacrylate copolymer are preferably used as a dispersing agent.

[Viscosity of composition]

The viscosity of the composition is preferably 100000 mPa·s or less. When the viscosity of the composition is 100000 mPa·s or less, the user can get a sense of what it is like to use a skin care product. When the viscosity of the composition is 100 mPa·s or less, the lipophilic porous powder easily precipitates, and thus the viscosity is preferably more than 100 mPa·s.

[Other]

In the component of the present invention, in addition to the above-described essential components, the components normally used in cosmetics or quasi-drug component can be blended, and they are produced according to conventional methods. In the following, specific blendable components are listed. The component of the present invention can be prepared by blending the above-described essential components and one or more of the below-described components.

Examples of moisturizers include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, sodium lactate, bile salts, dl-pyrrolidone carboxylates, short-chain soluble collagen, diglycerin (EO)PO adduct, chestnut rose extract, yarrow extract, and melilot extract.

Examples of powder components include inorganic powder (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate, calcium phosphate, fluorine apatite, hydroxyapatite, ceramic powder, metallic soap (for example, zinc myristate, calcium palimitate, and aluminum stearate), and boron nitride); organic powder (for example, polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, and cellulose powder); inorganic white family pigment (for example, zinc oxide); inorganic red family pigment (for example, iron titanate); inorganic purple family pigment (for example, mango violet, cobalt violet); inorganic green family pigment (for example, chrome oxide, chrome hydroxide, cobalt titanate); inorganic blue family pigment (for example, ultramarine, iron blue); pearl pigment (for example, titanium oxide coated mica, titanium oxide coated bismuth oxychloride, titanium oxide coated talc, colored titanium oxide coated mica, bismuth oxychloride, argentine); metal powder pigment (for example, aluminum powder, and copper powder); organic pigment such as zirconium, barium, or aluminum lake (for example, organic pigment such as Red No.201, Red No.202, Red No.204, Red No.205, Red No.220, Red No.226, Red No.228, Red No.405, Orange No.203, Orange No.204, Yellow No.205, Yellow No.401, Blue No.404,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); natural pigment (for example, chlorophyll, and β-carotene).

However, when an inorganic powder having a high refractive index (such as a refractive index of 2 or higher) is blended, the inorganic powder is preferably 5 mass % or less of the composition, and more preferably 1 mass % or less.

Examples of liquid fats include avocado oil, camellia oil, turtle oil, macadamia nut oil, corn oil, mink oil, olive oil, rapeseed oil, egg yolk oil, sesame oil, par chic oil, wheat germ oil, sasanqua oil, castor oil, linseed oil, safflower oil, cotton seed oil, perilla oil, soybean oil, groundnut oil, brown real oil, torreya oil, rice bran oil, chinese wood oil, jojoba oil, germ oil, and triglycerol.

Examples of solid fats include cacao butter, coconut oil, horse fat, hydrogenated coconut oil, palm oil, beef fat, mutton suet, hydrogenated beef fat, palm kernel oil, lard, beef bones fat, Japan wax kernel oil, hardened oil, hoof oil, Japan wax, and hydrogenated caster oil.

Examples of waxes include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of hydrocarbon oils include liquid paraffin, ozocerite, squalene, pristane, paraffin, ceresin, squalane, vaseline, and microcrystalline wax.

Examples of higher fatty acids include lauric acid, myristic acid, palmitic acid, stearic acid, behenic acid, oleic acid, undecylenic acid, tallic acid, isostearic acid, linoleic acid, linolenic acid, eicosapentaenoic acid(EPA), and docosahexaenoic acid(DHA).

Examples of higher alcohols include linear alcohol (for example, lauryl alcohol, cetyl alcohol, stearyl alcohol, behenyl alcohol, myristyl alcohol, oleyl alcohol, and cetostearyl alcohols); branched-chain alcohols (for example, monostearylglycerin ether (batyl alcohol),2-decyltetradecinol, lanolin alcohol, cholesterol, phytosterol, hexyldodecanol, isostearyl alcohol, and octyldodecanol).

Further, various surfactants may be blended into the composition of the present invention.

Examples of anionic surfactants include fatty acid soap (for example, sodium laurate, and sodium palmitate); higher alkyl sulfate ester salt (for example, sodium lauryl sulfate, and potassium lauryl sulfate); alkyl ether sulfate ester salt (for example, POE-lauryl sulfate triethanolamine, and sodium POE-lauryl sulfate); N-acyl sarcosinic acid (for example, sodium lauroyl sarcocinate); higher fatty acid amide sulfonate (for example, sodium N-myristoyl-N-methyl taurine, sodium coconut oil fatty acid methyl tauride, and sodium laurylmethyl tauride); phosphate ester salt (sodium POE-oleylether phosphate, and POE-stearylether phosphate); sulfosuccinate (for example, sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (for example, sodium linear dodecylbenzene sulfonate, triethanolamine linear dodeylbenzene sulfonate, and linear dodecylbenzene sulfonate); higher fatty acid ester sulfate ester salt (for example, sodium hydrogenated gryceryl cocoate sulfate); N-acyl glutamate (for example, monosodium N-lauroyl glutamate, disodium N-stearoyl glutamate, and monosodium N-myristoyl-L-glutamate); sulfonated oil (for example, Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; and sodium casein.

Examples of cationic surfactants include alkyltrimethyl ammonium salt (for example, stearyltrimethyl ammonium chloride, and lauryltrimethyl ammonium chloride); alkylpyridinium salt (for example, cetylpyridinium chloride); distearyldimethyl ammonium chloride; dialkyldimethyl ammonium salt; poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE-alkylamine; alkylamine salt; polyamine fatty acid derivative; amyl alcohol fatty acid derivative; benzalkonium chloride; and benzethonium chloride.

Examples of ampholytic surfactants include imidazoline base ampholytic surfactant (for example, sodium 2-undecyl-N,N,N-(hydroxyethylcarboxymethyl)-2-imidazoline, 2-cocoyl-2-imidazolinium hydroxide-1-carboxyethyloxy)-2-sodium salt; and betaine base surfactant (for example, 2-heptadecyl-N-carboxymethyl-N-hydroxyethylimidazolinium betaine, lauryldimethyl aminoacetate betaine, alkyl betaine, amidobetaine, and sulfobetaine).

Examples of lipophilic nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, and diglycerol sorbitan tetra-2 ethylhexylate); glyceryl polyglyceryl fatty acids (for example, glyceryl monocotton oil fatty acid, glyceryl monoerucate, glyceryl sesquioleate, glyceryl monostearate, glyceryl a, α'-oleate pyroglutamate, and glyceryl monostearate malate); propylene glycol fatty acid esters (for example, propylene glycol monostearate); hydrogenated caster oil derivative; and glyceryl alkyl ether.

Examples of hydrophilic nonionic surfactants include POE-sorbitan fatty acid esters (for example, POE-sorbitan monooleate, POE-sorbitan monostearate, and POE-sorbitan tetraoleate); POE-sorbit fatty acid esters (for example, POE-sorbit monolaurate, POE-sorbit monooleate, POE-sorbit pentaoleate, and POE-sorbit monostearate), POE-glyceryl fatty acid esters (for example, POE-glyceryl monostearate; POE-glyceryl monoisostearate, and POE-glyceryl triisostearate); POE-fatty acid esters (for example, POE-distearate, POE-monodioleate, and ethyleneglycol distearate); POE-alkyl ethers (for example, POE-lauryl ether, POE-oleyl ether, POE-stearyl ether, POE-behenyl ether, POE-2-octyldodecyl ether, and POE-cholestanol ether); pluronic types (for example, Pluronic), POE/POP-alkyl ethers (for example, POE/POP-cetyl ether, POE/POP-2-decyltetradecyl ether, POE/POP-monobutyl ether, POE/POP-hydrogenated lanoline, and POE/POP glycerin ether); tetra POE/tetra POP-ethylenediamine condensation products (for example, Tetronic); POE-castor oil hydrogenated castor oil derivatives (for example, POE-caster oil, POE-hydrogenated caster oil, POE-hydrogenated caster oil monoisostearate, POE-hydrogenated castor oil triisostearate, POE-hydrogenated caster oil monopyroglutamate monoisostearate diester, and POE-hydrogenated oil maleate); POE-beeswax/lanoline derivatives (for example, POE-sorbitol beeswax); alkanolamides (for example, coconut oil fatty acid diethanolamide, lauric acid monoethanolamide, and fatty acid isopropanolamide); POE-propyleneglycol fatty acid esters; POE-alkyl amines; POE-fatty acid amides; sucrose fatty acid esters; alkylethoxydimethylamine oxide; and trioleyl phosphoric acid.

When a low-molecular surfactant is used in the present invention, the low-molecular surfactant is preferably 1 mass % or less of the composition because the low-molecular surfactant is absorbed to the porous powder and may affect lipophilicity inside and outside of the powder.

Examples of natural water-soluble polymers include plant-based polymer (for example, gum Arabic, gum tragacanth, galactan, guar gum, locust bean gum, gum karaya, carrageenan, pectine, agar, quince seed (cydonia oblonga), algae colloid (brown algae extract), starch (rice, corn, potato, and wheat), and glicyrrhizic acid), microorganisms based polymer (for example, xanthan gum, dextran, succinoglycan, and pullulan), animal-based polymer (for example, collagen, casein, and albumin, gelatine).

Examples of semisynthetic water-soluble polymers include starch-based polymer (for example, carboxymethyl starch, and methylhydroxypropyl starch), cellulosic polymer (methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, micrclrystalline cellulose, and cellulose powder), and algin acid base polymer (for example, sodium alginate, and propylene glycol ester alginate).

Examples of synthetic water-soluble polymers include vinyl base polymer (for example, polyvinyl alcohol, polyvinyl methyl ether, polyvinylpyrrolidone, and carboxyvinylpolymer); polyoxyethylene base polymer (for example, polyethylene glycol 20,000, 40,000, and 60,000); acrylic polymer (for example, sodium polyacrylate, polyethylacrylate, and polyacrylamide); polyethyleneimine; and cationpolymer.

Examples of plasticizers except the following synthetic water-soluble polymers include, for example, dextrine, sodium pectate, sodium alginate, dialkyldimethylammonium cellulose sulfate, aluminium magnesium silicate, bentonite, hectorite, aluminium magnesium silicate (veegum), laponite, and silicic anhydride.

Examples of ultraviolet light absorbers include benzoic acid family ultraviolet light absorbers (for example, p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid family ultraviolet light absorbers (for example, homomenthyl N-acetylanthranilate); salicylic acid family ultraviolet light absorbers (for example, amyl salicylate, menthyl salicylate, homomenthyl salicylate, octyl salicylate, phenyl salicylate, benzyl salicylate, and p-isopropanolphenyl salicylate); cinnamic acid family ultraviolet light absorbers (for example, octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, and glyceryl mono-2-ethylhexanoyl-diparamethoxy cinnamate); benzophenone family ultraviolet light absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,1-camphor, 3-benzylidene-d,l-camphor; 2-phenyl-5-methylbenzoxazol; 2,2'-hydroxy-5-methylphenylbenzotriazol; 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; and 5-(3,3-dimethyl-2-norbornylidene)-3-pentane-2-one.

Examples of lower alcohols include ethanol, propanol, isopropanol, isobutyl alcohol, t-butyl alcohol.

Examples of polyhydric alcohols include dihydric alcohol (for example, ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol); trihydric alcohol (for example, glycerin, and trimethylolpropane); tetrahydric alcohol (for example, pentaerythritol such as 1,2,6-hexanetriol); pentahydric alcohol (for example, xylitol); hexahydric alcohol (for example, sorbitol, and mannitol); polyhydric alcohol polymer (for example, diethylene glycol, triethylene glycol, polypropylene glycol, tetraethylene glycol, diglycerin, polyethylene glycol, triglycerin, tetraglycerin, and polyglycerin); dihydric alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether ethers (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin monoalkyl ether (for example, chimil alcohol, selachyl alcohol, and batyl alcohol); sugar alcohol (for example, sorbitol, maltitol, maltotriose, mannitol, sucrose, erythritol, glucose, fructose, starch sugar, maltose, xylitose, and starch sugar hydrogenated alcohol); glysolid; tetrahydrofurfuryl alcohol; POE-tetrahydrofurfuryl alcohol; POP-butyl ether; POP/POE-butyl ether; tripolyoxypropylene glycerin ether; POP- glycerin ether; POP-glycerin ether phosphoric acid; POP/POE-pentaerythritol ether; and polyglycerin.

Examples of monosaccharides include triose (for example, D-glyceryl aldehyde, and dihydroxyacetone); tetrose (for example, D-erythrose, D- erythrulose, D-threose, and erythritol); pentaose (for example, L-arabinose, D-xylose, L-lyxose, D-arabinose, D-ribose, D-ribulose, D-xylulose, and L-xylulose); hexalose (for example, D-glucose, D-talose, D-psicose, D-galactose, D-fructose, L-galactose, L-mannose, and D-tagatose); heptose (for example, aldoheptose, and heplose); octose (for example, octulose); deoxy sugar (for example, 2-deoxy-D-ribose, 6-deoxy-L- galactose, and 6-deoxy-L-mannose); amino sugar (for example, D-glucosamine, D-galactosamine, sialic acid, amino uronic acid, and muramic acid); and uronic acid (for example, D-grucuronic acid, D-mannuronic acid, L-guluronic acid, D-garacturonic acid, and L-iduronic acid).

Examples of oligosaccharides include sucrose, guntianose, umbelliferose, lactose, planteose, isolignoses, $\alpha,\alpha$-trehalose, raffinose, lignoses, umbellicine, stachyose, and verbascoses.

Examples of amino acids include neutral amino acid (for example threonine, and cysteine); and basic amino acid (for example, hydroxylysine). Examples of amino acid derivatives include sodium acyl sarcosine (sodium lauroyl sarcosine), acyl glutamate, sodium acyl $\beta$-alanine, glutathione, and pyrrolidone carboxylate.

Examples of the organic amines include monoethanolamine, diethanolamine, triethanolamine, morpholine, triisopropanolamine, 2-amino-2-methyl-1,3-propanediol, and 2-amino-2-methyl-1-propanol.

Example of the alkylene oxide derivatives include POE (9) POP (2) dimethyl ether , POE (14) POP (7) dimethyl ether, POE (10) POP (10) dimethyl ether, POE (6) POP (14) dimethyl ether, POE (15) POP (5) dimethyl ether, POE (25) POP (2 5) dimethyl ether, POE (7) POP (12) dimethyl ether, POE (2 2) POP (40) dimethyl ether, POE (3 5) POP (40) dimethyl ether, POE (50) POP (40) dimethyl ether, POE (55) POP (30) dimethyl ether, POE (30) POP (34) dimethyl ether, POE (25) POP (30) dimethyl ether, POE (27) POP (14) dimethyl ether, POE (55) POP (28) dimethyl ether, POE (36) POP (41) dimethyl ether, POE (7) POP (12) dimethyl ether and POE (17) POP (4) dimethyl ether.

Examples of chelate agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of anti-oxidant aids include phosphoric acid, citric acid, ascorbic acid, maleic acid, malonic acid, succinic acid, fumaric acid, cephalin, hexamethaphosphate, phytic acid, and ethylene diamine tetra-acetic acid.

Examples of other blendable components include antiseptic agent (ethylparaben, butylparaben, etc); lightening agent (for example, placental extract, saxifrage extract, and arbutin); blood circulation promotion agent (for example, nicotine acid, nicotine acid benzyl, tocopherol nicotinate, nicotine acid $\beta$-butoxy ester, minoxidil, or their analogs, vitamine E type, $\gamma$-oryzanol, alkoxycarbonylpyridine N-oxide, capronium chloride, acetylcholine and their derivatives); various extract (for example, ginger, oat, Japanese coptis, lithospermum, birch, loquat, carrot, aloe, mallow, iris, grape, sponge gourd, lily, saffron, cnidium rhizome, ginger , hypericum, restharrow, garlic, red pepper, citrus unshiu, Japanese angelica, Japanese tree peoney, and seaweed); activator agent (for example, pantothenyl ethyl ether, nicotinamide, biotin, pantothenic acid, royal jelly, and cholesterol derivative); and antiseborrheric agent (for example, pyridoxine, and thianthl).

The compsition of the present invention can be in any form, and the examples include a solution form, emulsion form,, lotion, gel, mist, spray and mousse.

Hereinafter, preferred embodiments of the present invention will be described in detail.

First, test methods will be described.

(Dispersed state)

The dispersed state of prepared samples was observed under a microscope. In the field of view at a magnification of 100×, a state in which no aggregate was found was defined as A, a state in which 10 or less aggregates were found was defined as B, a state in which 50 or less aggregates were found was defined as C, and a state in which more than 50 aggregates were found was defined as D. A and B were each regarded as a good dispersed state.

(Stability)

The samples were each kept in a 50-ml glass bottle at room temperature for four months and then the state of each sample was visually observed. A state in which no separation was found was defined as A, a state in which a transparent layer was slightly found was defined as B, a state in which a transparent layer was found in about one tenth or less of the whole was defined as C, and a state in which a transparent layer was found in a range wide than that of C was defined as D. However, even if the stability of a sample having a good dispersed state is poor, the sample can be used without any problems by shaking the sample well before use.

(Pore-correcting effect)

Professional panelists (10 members) used the samples and considered a sample having a good pore-correcting effect to be A, a sample having a slight pore-correcting effect to be B, a sample having a poor pore-correcting effect to be C, and a sample having no pore-correcting effect to be D. A and B were each regarded as having a good pore-correcting effect.

(Whiteness)

Professional panelists (10 members) used samples and considered a sample having completely unnoticeable whiteness to be A, a sample having a slight but hardly noticeable whiteness to be B, a sample having noticeable whiteness to be C, and a sample having too much and obviously unnatural whiteness to be D.

(Powderiness)

Professional panelists (10 members) used samples and considered a sample having little powderiness to be A, a sample having slight powderiness to be B, and a sample having considerable powderiness to be C.

(Viscosity)

Each sample was measured using a BL-type viscometer (manufactured by Shibaura Semtek Co., Ltd.) with a rotor No.2 at a revolution speed of 12 rpm for 60 seconds at 30°C.

Instead of conditioning defects on the skin such as correcting pores using the covering effect of an inorganic powder as found using commonly used foundations or the like, the present inventors used a lipophilic porous powder which was an organic (resin) powder having relatively high transparency fall into depressed parts such as pores, and the light diffusion effect of resin powder make the depressed parts unnoticeable.

Such a resin powder has relatively high transparency and also has a refractive index similar to that of a cosmetic base, and thus can improve the transparency of the composition, but in order to improve the water resistance of the composition, the resin powder needs to be selected.

Additionally, the resin powder, particularly a spherical resin powder, provides a good impression during use but tends to provide a slightly low light diffusion effect, and for that reason, use of a porous powder is expected to improve the light diffusion effect.

Then, the present inventors conducted tests as shown in the following Table 1, and the pore-correcting effect of the resin powders used as the lipophilic porous powder was studied. In Tables 1 to 8, the content of each raw material used in the composition is in % by mass.

[table 1]

| | | 1-1 | 1-2 | 1-3 | 1-4 |
|---|---|---|---|---|---|
| dispersion medium | Ion exchanged water | balance | balance | balance | balance |
| | Glycerine | 10 | 10 | 10 | 10 |
| | Butyleneglycol | 10 | 10 | 10 | 10 |
| | | | | | |
| oil | Glyceryl tri-2-ethyl hexanoate | 15 | | 15 | 15 |
| | | | | | |
| powder | Crosslinked polymethylmethacrylate porous powder | | 15 | 15 | |
| | Crosslinked polymethylmethacrylate non-porous powder | | | | 15 |
| | | | | | |
| Dispersing agent | Acrylic acid-methacrylic acid alkyl copolymer | 0.05 | 0.05 | 0.05 | 0.05 |
| | | | | | |
| other | Sodium Metaphosphate | 0.01 | 0.01 | 0.01 | 0.01 |
| | Para-hydroxybenzonate | 0.1 | 0.1 | 0.1 | 0.1 |
| | Sodium hydroxide | 0.025 | 0.025 | 0.025 | 0.025 |
| | | | | | |
| valuation | dispersion state | A | A | A | A |
| | pore-correcting effect | D | A | A | C |
| | whiteness | A | D | A | A |

As shown in Table 1, when no resin powder was blended (Test Example 1-1), almost no pore-correcting effect was provided. On the other hand, when a non-porous resin powder was blended (Test Example 1-4), the pore-correcting effect was provided but at an insufficient level.

In contrast, when the porous powder was blended (Test Examples 1-2 and 1-3), a high pore-correcting effect was noted, but when only the porous powder was blended into an aqueous dispersion medium (Test Example 1-2), the porous powder floated and showed poor dispersibility, and additionally, upon application to the skin the light scattering effect was too strong that the skin looked white.

On the other hand, when the lipophilic porous powder (the crosslinked polymethyl methacrylate porous powder used in the Test Examples of the present invention had a glyceryl tri-2-ethylhexanoate oil absorption of 210%) was allowed to absorb the oil (Test Example 1-3), scattering of light was moderately reduced and the apparent specific gravity of the resin powder was also controlled, and thus the dispersibility was also good.

Next, the present inventors conducted a study of the types of powders. Results are shown in Table 2.

[table 2]

| | | 2-1 | 2-2 | 2-3 | 2-4 |
|---|---|---|---|---|---|
| Dispersion medium | Ion exchanged water | balance | balance | balance | balance |
| | Glycerine | 10 | 10 | 10 | 10 |
| | Butyleneglycol | 10 | 10 | 10 | 10 |
| | | | | | |
| Oil | Glyceryl tri-2-ethyl hexanoate | 15 | 15 | 15 | 15 |
| | | | | | |

(continued)

|  |  | 2-1 | 2-2 | 2-3 | 2-4 |
|---|---|---|---|---|---|
| Powder | Crosslinked polymethylmethacrylate porous powder (maximum oil absorption 210%) | 15 |  |  |  |
|  | Nylon porous powder(maximum oil absorption 105%) |  | 15 |  |  |
|  | Cellulose powder(maximum oil absorption 100%) |  |  | 15 |  |
|  | Porous silica(maximum oil absorption 145%) |  |  |  | 15 |
|  |  |  |  |  |  |
| Dispersing agent | Acrylic acid-methacrylic acid alkyl copolymer | 0.05 | 0.05 | 0.05 | 0.05 |
|  |  |  |  |  |  |
| Other | Sodium Metaphosphate | 0.01 | 0.01 | 0.01 | 0.01 |
|  | Para-hydroxybenzonate | 0.1 | 0.1 | 0.1 | 0.1 |
|  | Sodium hydroxide | 0.025 | 0.025 | 0.025 | 0.025 |
|  |  |  |  |  |  |
| Valuation | dispersion state | A | A | A | A |
|  | pore-correcting effect | A | A | A | C |
|  | whiteness | A | A | A | A |

As shown in Table 2, in addition to the crosslinked polymethyl methacrylate porous powder which provided a high pore-correcting effect, a porous nylon powder (Test Example 2-1) and a porous cellulose powder (Test Example 2-2) also provided a high pore-correcting effect, and also had a good dispersed state and provided appropriate whiteness. When a hydrophilic porous silica (Test Example 2-3) was used, no good pore-correcting effect was obtained.

In the following, the inventor investigated the oil.

Results are shown in Table 3.

[table 3]

| | | 1-3 | 3-1 | 3-2 | 3-3 | 3-4 | 3-5 |
|---|---|---|---|---|---|---|---|
| Dispersion medium | Ion exchanged water | balance | balance | balance | balance | balance | balance |
| | Glycerine | 10 | 10 | 10 | 10 | 10 | 10 |
| | Butyleneglycol | 10 | 10 | 10 | 10 | 10 | 10 |
| Oil | Glyceryl tri-2-ethyl hexanoate (28mPa·s) | 15 | | | | | |
| | Dimethyl silicone (6mPa·s) | | 15 | | | | |
| | Dimethyl silicone (100mPa·s) | | | 15 | | | |
| | Methylphenyl silicone (6mPa·s) | | | | 15 | | |
| | Liquid faraffin (6mPa·s) | | | | | 15 | |
| | Malic acid diisostearyl (1700mPa·s) | | | | | | 15 |
| Powder | Crosslinked polymethyl methacrylate porous powder(maximum oil absorption 210%) | 15 | 15 | 15 | 15 | 15 | 15 |
| Dispersing agent | Acrylic acid-methacrylic acid alkyl copolymer | 0.05 | 0.05 | 0.05 | 0.05 | 0.05 | 0.05 |
| Other | Sodium Metaphosphate | 0.01 | 0.01 | 0.01 | 0.01 | 0.01 | 0.01 |
| | Para-hydroxybenzonate | 0.1 | 0.1 | 0.1 | 0.1 | 0.1 | 0.1 |
| | Sodium hydroxide | 0.025 | 0.025 | 0.025 | 0.025 | 0.025 | 0.025 |
| Valuation | dispersion state | A | A | Indeterminable | A | A | smudge |
| | pore-correcting effect | A | A | D | A | A | Indeterminable |
| | viscosity | 2830 | 66300 | 7400 | 49300 | 78700 | 4430 |

Based on the results shown in Table 3, ester oils, silicone oils, hydrocarbon oils, and the like were considered as the oil, and when the viscosity of the oil was 100 mPa·s or more (Test Examples 3-2 and 3-5), it was considered that the oil was unlikely to be absorbed by the powder, and no pore-correcting effect was note. In contrast, when the viscosity of the oil is less than 100 mPa·s, even the ester oil, the silicone oil, and the hydrocarbon oil provided a good pore-correcting effect, and when the silicone oil was used (Test Examples 3-1 and 3-3) or when the hydrocarbon oil was used (Test Example 3-4), the viscosity tended to increase, but the pore-correcting effect and the whitening effect were noted.

Accordingly, if an impression during use is intended to be similar to that of a essence or a milky lotion which are commonly used skin care cosmetics, a low-viscosity ester oil is preferably used so that the composition has a viscosity of 10000 mPa·s or less.

Further, if an impression during use is intended to be similar to that of gel-form cosmetics, a silicone oil or a hydrocarbon oil is preferably used so that the viscosity is 10000 mPa·s or more.

The present inventors proceeded with studying mainly ester oils having a viscosity of 100 mPa·s or less. Results are shown in Table 4.

[table 4]

|  |  | 4-1 | 4-2 | 4-3 | 4-4 | 4-5 |
|---|---|---|---|---|---|---|
| Dispersion medium | Ion exchanged water | balance | balance | balance | balance | balance |
|  | Glycerine | 10 | 10 | 10 | 10 | 10 |
|  | Butyleneglycol | 10 | 10 | 10 | 10 | 10 |
|  |  |  |  |  |  |  |
| Oil | Glyceryl tri-2-ethyl hexanoate (28mPa·s) | 12 |  |  |  |  |
|  | Pentaerythrityl tetra-2-ethyl hexanoate (70mPa·s) |  | 12 |  |  |  |
|  | 2-Cetyl Ethylhexanoate (70mPa·s) |  |  | 12 |  |  |
|  | Neo pentansan isodeshiru (8.5mPa·s) |  |  |  | 12 |  |
|  | 2-ethylhexyl succinate (22mPa·s) |  |  |  |  | 12 |
|  |  |  |  |  |  |  |
|  |  |  |  |  |  |  |
| Powder | Crosslinked polymethyl methacrylate porous powder(maximum oil absorption 210%) | 12 | 12 | 12 | 12 | 12 |
|  |  |  |  |  |  |  |
| Dispersing agent | Acrylic acid-methacrylic acid alkyl copolymer | 0.05 | 0.05 | 0.05 | 0.05 | 0.05 |
|  |  |  |  |  |  |  |
| Other | Sodium Metaphosphate | 0.01 | 0.01 | 0.01 | 0.01 | 0.01 |
|  | Para-hydroxybenzonate | 0.1 | 0.1 | 0.1 | 0.1 | 0.1 |
|  | Sodium hydroxide | 0.025 | 0.025 | 0.025 | 0.025 | 0.025 |
|  |  |  |  |  |  |  |
| Valuation | pore-correcting effect | A | B | A | A | A |
|  | whitness | B | B | A | B | B |
|  | viscosity | 2330 | 4760 | 7100 | 5760 | 2980 |

Based on Table 4, when a composition for skin care is prepared, glyceryl tri-2-ethylhexanoate or di-2-ethylhexyl succinate is preferably used.

The present inventors conducted a study of the blending ratio of the ester oil and the lipophilic porous powder. The result indicates Table5.

In Table 5, X refers to the maximum oil absorption (% by mass) of the lipophilic porous powder and Y refers to (Oil content (% by mass) of composition) $\times$ 100)/(Lipophilic porous powder content (% by mass) of composition).

[table 5]

| | | 5-1 | 5-2 | 5-3 | 5-4 | 5-5 | 5-6 | 5-7 | 5-8 |
|---|---|---|---|---|---|---|---|---|---|
| Dispersion medium | Ion exchanged water | balance | balance | balance | balance | balance | balance | balance | balance |
| | Glycerine | 10 | 10 | 10 | 10 | 10 | 10 | 10 | 10 |
| | Butyleneglycol | 8 | 8 | 8 | 8 | 8 | 8 | 8 | 8 |
| Oil | Glyceryl tri-2-ethyl hexanoate (28mPa·s) | 3 | 5 | 8 | 10 | 14 | 17 | 20 | 23 |
| Powder | Crosslinked polymethyl methacrylate porous powder (maximum oil absorption 210%) | 10 | 10 | 10 | 10 | 10 | 10 | 10 | 10 |
| Dispersing agent | Acrylic acid-methacrylic acid alkyl copolymer | 0.03 | 0.03 | 0.03 | 0.03 | 0.03 | 0.03 | 0.03 | 0.03 |
| Other | Sodium metaphosphate | 0.01 | 0.01 | 0.01 | 0.01 | 0.01 | 0.01 | 0.01 | 0.01 |
| | Ethyl p-hydroxybenzoate | 0.1 | 0.1 | 0.1 | 0.1 | 0.1 | 0.1 | 0.1 | 0.1 |
| | Methyl p-hydroxybenzoate | 0.15 | 0.15 | 0.15 | 0.15 | 0.15 | 0.15 | 0.15 | 0.15 |
| | Phenoxyethanol | 0.5 | 0.5 | 0.5 | 0.5 | 0.5 | 0.5 | 0.5 | 0.5 |
| | Synthetic hydrocarbon waxes | 4 | 4 | 4 | 4 | 4 | 4 | 4 | 4 |
| | Hydroxyethyl Acrylate/Sodium Acryloyldimethyl Taurate Coporymer | 0.65 | 0.65 | 0.65 | 0.65 | 0.65 | 0.65 | 0.65 | 0.65 |
| | polyvinyl alcohol | 0.1 | 0.1 | 0.1 | 0.1 | 0.1 | 0.1 | 0.1 | 0.1 |
| | sodium hydroxide | 0.015 | 0.015 | 0.015 | 0.015 | 0.015 | 0.015 | 0.015 | 0.015 |
| | Y | 30 | 50 | 80 | 100 | 140 | 170 | 200 | 230 |
| | Y/X | 0.14 | 0.23 | 0.38 | 0.48 | 0.67 | 0.81 | 0.95 | 1.1 |
| Valuation | pore-correcting effect | A | A | A | A | A | A | B | D |
| | whiteness | A | A | A | A | A | A | A | A |

As is obvious from Table 5, when the oil content is up to about 200% by mass based on the lipophilic porous powder, a good pore-correcting effect can be obtained and an appropriate whiteness can be obtained. However, when the oil content reaches 200% by mass, no pore-correcting effect is provided.

Accordingly, the oil content is 30 to 200% by mass, particularly preferably 170% by mass or less, based on the porous powder. That is, Y/X is preferably 1 or less.

The present inventors conducted a study of a combined use of the lipophilic porous powder which is characteristic of the present invention and a powder other than the lipophilic porous powder. Results are shown in Table6.

[table 6]

| | | 6-1 | 6-2 | 6-3 |
|---|---|---|---|---|
| Dispersion medium | Ion exchanged water | balance | balance | balance |
| | Glycerine | 10 | 10 | 10 |
| | Butyleneglycol | 10 | 10 | 10 |
| | | | | |
| Oil | Glyceryl tri-2-ethyl hexanoate | 12 | 12 | 12 |
| | | | | |
| Powder | Crosslinked polymethylmethacrylate porous powder(maximum oil absorption 210%) | 12 | 12 | 12 |
| | (dimeticon/vinyldimeticon) Crosspolymer (maximum oil absorption 165%) | 8 | | |
| | Porous silica(maximum oil absorption 145%) | | 5 | |
| | Nylon powder(maximum oil | | | 5 |
| | absorption 105%) | | | |
| | | | | |
| Dispersing agent | Acrylic acid-methacrylic acid alkyl copolymer | 0.05 | 0.05 | 0.05 |
| | | | | |
| Other | Sodium Metaphosphate | 0.01 | 0.01 | 0.01 |
| | Para-hydroxybenzonate | 0.1 | 0.1 | 0.1 |
| | Sodium hydroxide | 0.025 | 0.025 | 0.025 |
| | | | | |
| Valuation | pore-correcting effect | A | A | A |
| | whiteness | A | B | A |
| | viscosity | 20900 | 2330 | 2120 |

It is understood from Table 6 that even though a powder other than the lipophilic porous powder coexists with the lipophilic porous powder, such a combined use does not particularly adversely affect the pore-correcting effect and whiteness which are characteristic of the present invention.

The present inventors conducted a study of dispersing agents for the lipophilic porous powder. Results are shown in Table7.

[table 7]

| Category | | | 7-1 | 7-2 | 7-3 | 7-4 | 7-5 | 7-6 | 7-7 |
|---|---|---|---|---|---|---|---|---|---|
| Dispersion medium | | Ion exchanged water | balance | balance | balance | balance | balance | balance | balance |
| | | Glycerine | 10 | 10 | 10 | 10 | 10 | 10 | 10 |
| | | Butyleneglycol | 10 | 10 | 10 | 10 | 10 | 10 | 10 |
| Oil | | Glyceryl tri-2-ethyl hexanoate | 5 | 5 | 5 | 5 | 5 | 5 | 5 |
| | | Diethylhexyl Succinate | 4 | 4 | 4 | 4 | 4 | 4 | 4 |
| | | Cetyl Ethylhexanoate (70mPa ∙ s) | 1 | 1 | 1 | 1 | 1 | 1 | 1 |
| Powder | | Crosslinked polymethyl methacrylate porous powder | 10 | 10 | 10 | 10 | 10 | 10 | 10 |
| | | Cellulose powder | 3 | 3 | 3 | 3 | 3 | 3 | 3 |
| Dispersing agent | | Acrylic acid-methacrylic acid alkyl copolymer | 0.05 | 0.1 | | | | 0.02 | 0.05 |
| | | Ammonium Acryloyldimethyltaurate/VP Copolymer | | | 0.2 | | | | |
| | | Hydroxyethyl Acrylate/Sodium Acryloyldimethyl Taurate Copolymer | | | | 0.5 | | 0.5 | |
| | | Dimethylacrylamide/Sodium Acryloyldimethyltaurate Crosspolymer | | | | | 0.15 | 0.15 | 0.15 |
| Other | | Sodium Metaphosphate | 0.01 | 0.01 | 0.01 | 0.01 | 0.01 | 0.01 | 0.01 |
| | | Para-hydroxybenzonate | 0.1 | 0.1 | 0.1 | 0.1 | 0.1 | 0.1 | 0.1 |
| | | Sodium hydroxide | appropriate | appropriate | appropriate | appropriate | appropriate | appropriate | appropriate |

16

| | | 7-1 | 7-2 | 7-3 | 7-4 | 7-5 | 7-6 | 7-7 |
|---|---|---|---|---|---|---|---|---|
| Valuation | pore-correcting effect | A | A | B | A | A | A | A |
| | whiteness | A | A | A | A | A | A | A |
| | viscosity | 1240 | 6850 | 7610 | 1230 | 1380 | 2180 | 6010 |
| | dispersion state | A | A | C | C | C | A | A |
| | stability | D | A | D | B | B | A | A |

According to Table 7, when a polymer dispersing agent, particularly a carboxylic acid-based agent alone such as an acrylic acid/alkyl methacrylate copolymer was used or a carboxylic acid-based agent was contained as the dispersing agent for the hydrophobic porous powder, the dispersed state was good and the temporal stability was also obtained. In Test Example 7-1, the stability was rated as D, but the precipitation of the powder can be eliminated by shaking the composition. In Test Examples (7-3, 7-4, and 7-5) in which a sulfonic acid-based agent alone was used, the ratings of the pore-correcting effect and the whiteness were good. When the sulfonic acid-based agent alone was used, the dispersed state easily becomes poor.

The present inventors tried to further improve the impression during use by adding a wax particulate dispersion.

That is, the wax particulate dispersion in which wax particulates (having a particle diameter of approximately 10 $\mu$m) were dispersed in an aqueous medium was added to conduct a study of improvement in the impression during use. Results are shown in Table 8.

[table 8]

| | | 7-1 | 8-1 | 8-2 | 8-3 | 8-4 | 8-5 | 8-6 |
|---|---|---|---|---|---|---|---|---|
| Dispersi on medium | Ion exchanged water | balance | balance | balance | balance | balance | balance | balance |
| | Glycerine | 10 | 10 | 10 | 10 | 10 | 10 | 10 |
| | Butyleneglycol | 10 | 10 | 10 | 10 | 10 | 10 | 10 |
| | | | | | | | | |
| Oil | Glyceryl tri-2-ethyl hexanoate | 5 | 5 | 5 | 5 | 5 | 5 | 5 |
| | Diethylhexyl Succinate | 4 | 4 | 4 | 4 | 4 | 4 | 4 |
| | Cetyl Ethylhexanoate (70 mPa ▪ s) | 1 | 1 | 1 | 1 | 1 | 1 | 1 |
| | | | | | | | | |
| Powder | Crosslinked polymethylmethacryl ate porous powder | 10 | 10 | 10 | 10 | 10 | 10 | 10 |
| | Cellulose powder | 3 | 3 | 3 | 3 | 3 | 3 | 3 |
| | | | | | | | | |
| Dispersi ng agent | Acrylic acid-methacrylic acid alkyl copolymer | 0.05 | 0.05 | 0.05 | 0.05 | 0.05 | 0.05 | 0.05 |
| | | | | | | | | |
| | Wax particle dispersion (wax 10%) | 0 | 5 | 10 | 12 | 15 | 20 | 28 |
| | | | | | | | | |
| Other | Sodium Metaphosphate | 0.01 | 0.01 | 0.01 | 0.01 | 0.01 | 0.01 | 0.01 |
| | Para-hydroxybenzon ate | 0.1 | 0.1 | 0.1 | 0.1 | 0.1 | 0.1 | 0.1 |
| | Sodium hydroxide | appropria te | appropria te | appropria te | appropria te | appropria te | appropria te | appropria te |
| | | | | | | | | |
| Valuatio n | pore-correcting effect | A | A | A | A | A | A | B |
| | whiteness | A | A | A | A | A | A | A |
| | viscosity | 2160 | 2210 | 1710 | 2120 | 2380 | 9680 | 18900 |
| | Powderiness | B | B | A | A | A | A | A |

As is obvious from the table, improvement in the impression during use (powderiness) was noted when the wax particle dispersion having an actual wax content of about 0.5 to 2.5% by mass was added, and it was noted that an actual wax content of nearly 3% by mass adversely affected the pore-correcting effect.

Accordingly, when the wax particulates are added, about 0.5 to 2.5% by mass of the wax particulates are preferably added.

The present inventors conducted a study of a production method.

First, when an oil component was mixed with the lipophilic porous powder and resulting mixture was dispersed in the aqueous medium, the powder remained aggregated regardless of whether or not the aqueous medium contained the dispersing agent. Additionally, also when the lipophilic porous powder was dispersed in the aqueous medium containing no dispersing agent and the oil was added to that aqueous medium, the powder aggregated.

On the other hand, when the lipophilic porous powder was dispersed in the aqueous medium containing the dispersing agent and the oil was then added to that aqueous medium, the oil was slowly kept by the lipophilic porous powder and eventually a homogeneous dispersion without aggregation was obtained.

Blending example 1

| | |
|---|---|
| Ion exchanged water | remainder |
| glycerine | 1 0 |
| Sodium Hexametaphosphate | 0.01 |
| 1,3-Butyleneglycol | 8 |
| Synthetic hydrocarbon waxes | 4 |
| Polyvinyl alcohol | 0.1 |
| Porous nylon powder | 1 0 |
| Acrylic acid-methacrylic acid alkyl copolymer | 0.03 |
| Triethylhexanoin | 10 |
| Potassium hydroxide | 0.015 |
| Hydroxyethyl Acrylate/Sodium Acryloyldimethyl Taurate Coporymer | 0.7 |
| Active ingredient * | proper quantity |

Example of active ingredient in Blending example 1 includes. Without impairing the effect of the present invention, The component of the present invention can be prepared by blending one or more of the below-described components.

Trehalose
Glycylglycine
Bergenia ciliate
Berugenia Syria data roots extract
Serine
Marjoram leaf extract
Horse chestnut seed extract
Erythritol
Lauroyl glutamate, di (phytosteryl / octyl pyrrolidone del
PEG/PPG-14/7 dimethyl ether
PEG/PPG-17/4 dimethyl ether
Sulfuric acid Ba
Rosemary leaf extract
Chamomile flower extract
Arginine
Saccharomyces lysate extract
Tranexamic acid
Mugwort leaf extract
Hamamelis leaf extract
Acetyl hyaluronate Na
Inositol
Water-soluble collagen

2-O-ethyl ascorbic acid Torumenchira root extract
Olive leaf extract
Lysine HCL
Hibiscus flower extract
Hydrolyzed silk
Glycyrrhizin acid 2K
Wild thyme extract
Tocopherol acetate
Gambir Extract
Panax ginseng root extract
Saxifrage extract
Bupleurum roots extract
Theanine
Suctellariabaicalensis roots extract
Mangosteen bark extract
Hydrolysis conchiolin
Allantoin

**Claims**

1. A composition, comprising:

   an aqueous medium;
   a lipophilic porous powder dispersed in the aqueous medium;
   an oil having a viscosity of 100 mPa·s or less, measured with the method disclosed in the present description, the oil being kept by at least some of pores of the lipophilic porous powder; and a dispersing agent for dispersing the lipophilic porous powder in the aqueous medium,
   wherein a maximum allowable oil absorption of the lipophilic porous powder, measured with the method disclosed in the present description, is 100% by mass or more based on the powder, and
   wherein the composition satisfies the following formula:

$$[(\text{Oil content (\% by mass) of composition})/ (\text{Lipophilic porous powder content (\% by mass)}$$
$$\text{of composition}) \times 100]/\text{Maximum oil absorption (\% by mass) of lipophilic porous powder} \leq 1.$$

2. The composition according to claim 1, wherein the lipophilic porous powder comprises crosslinked polymethyl methacrylate as a main material.

3. The composition according to claims 1 or 2, wherein a particulate wax is blended into the composition.

4. The composition according to claim 3, wherein a viscosity of the composition is 100000 mPa·s or less at 25°C.

5. Use of the composition according to any of claims 1 to 4 for correcting pores.

**Patentansprüche**

1. Zusammensetzung, umfassend:

   ein wässriges Medium;
   ein lipophiles poröses Pulver, das in dem wässrigen Medium dispergiert ist;
   ein Öl, das eine Viskosität von 100 mPa·s oder weniger aufweist, gemessen mit dem Verfahren, das in der vorliegenden Beschreibung dargelegt wird, wobei das Öl durch wenigstens einige Poren des lipophilen porösen Pulvers gehalten wird; und
   ein Dispergiermittel zum Dispergieren des lipophilen porösen Pulvers in dem wässrigen Medium, wobei eine maximal zulässige Ölabsorption des lipophilen porösen Pulvers, gemessen mit dem Verfahren, das in der vorliegenden Beschreibung dargelegt wird, 100 Masse-% oder mehr, basierend auf dem Pulver, beträgt und wobei die Zusammensetzung die folgende Formel erfüllt:

$$[(\text{Ölgehalt (Masse-\%) der Zusammensetzung})/(\text{Gehalt an lipophilem porösem Pulver (Masse-\%) der Zusammensetzung}) \times 100]/\text{Maximale Ölabsorption (Masse-\%) von lipophilem porösem Pulver} \leq 1.$$

**2.** Zusammensetzung nach Anspruch 1, wobei das lipophile poröse Pulver vernetztes Polymethylmethacrylat als Hauptmaterial umfasst.

**3.** Zusammensetzung nach Anspruch 1 oder 2, wobei ein teilchenförmiges Wachs in die Zusammensetzung beigemischt wird.

**4.** Zusammensetzung nach Anspruch 3, wobei eine Viskosität der Zusammensetzung 100000 mPa·s oder weniger bei 25 °C beträgt.

**5.** Verwendung der Zusammensetzung nach einem der Ansprüche 1 bis 4 zum Korrigieren von Poren.

**Revendications**

**1.** Composition comprenant :

un milieu aqueux ;
une poudre poreuse lipophile dispersée dans le milieu aqueux ;
une huile ayant une viscosité de 100 mPa·s ou moins, mesurée avec le procédé décrit dans la présente description, l'huile étant conservée par au moins certains des pores de la poudre poreuse lipophile ; et
un dispersant pour disperser la poudre poreuse lipophile dans le milieu aqueux,
une absorption d'huile maximale admissible de la poudre poreuse lipophile, mesurée avec le procédé décrit dans la présente description, étant de 100 % en masse ou plus sur la base de la poudre, et
la composition répondant à la formule suivante :

$$[(\text{Teneur en huile (\% en masse) de la composition}) / (\text{Teneur en poudre poreuse lipophile (\% en masse) de la composition}) \times 100] / \text{Absorption maximale en huile (\% en masse) de la poudre poreuse lipophile} \leq 1.$$

**2.** Composition selon la revendication 1, dans laquelle la poudre poreuse lipophile comprend du polyméthacrylate de méthyle réticulé comme matériau principal.

**3.** Composition selon les revendications 1 ou 2, dans laquelle une cire particulaire est mélangée à la composition.

**4.** Composition selon la revendication 3, dans laquelle une viscosité de la composition est de 100 000 mPa•s ou moins à 25 °C.

**5.** Utilisation de la composition selon l'une quelconque des revendications 1 à 4 pour corriger les pores.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0948723 A
- JP 2006063032 A
- JP 3291195 A

- JP H10203936 A
- JP H05201826 A